# EUROPÄISCHE PATENTSCHRIFT

(11) **EP 1 827 203 B1**
(45) Veröffentlichungstag und Bekanntmachung des Hinweises auf die Patenterteilung: **28.09.2011**
(21) Anmeldenummer: 05821465.1
(22) Anmeldetag: 20.12.2005
(51) Int. Cl.: A61B 3/16

(54) **MOBILES TONOMETER ZUR DURCHFÜHRUNG EINER BERÜHRUNGSFREIEN SELBSTTONOMETRIE**
MOBILE TONOMETER FOR CARRYING OUT CONTACTLESS AUTOMATIC TONOMETRY
TONOMETRE MOBILE SERVANT A REALISER UNE AUTO-TONOMETRIE SANS CONTACT

(30) Priorität: 20.12.2004 DE 102004062337
(43) Veröffentlichungstag der Anmeldung: 05.09.2007
(73) Patentinhaber: Mechatronic AG, 64295 Darmstadt (DE)
(72) Erfinder: WEBER, Thomas, 64291 Darmstadt (DE); BACKHAUS, Wendelin, 35789 Weilmünster (DE)
(74) Vertreter: Bill, Burkart Hartmut
(86) Internationale Anmeldenummer: PCT/EP2005/013712
(87) Internationale Veröffentlichungsnummer: WO 2006/066876

(56) Entgegenhaltungen:
- EP-A- 1 121 895
- WO-A1-98/09564
- US-A- 5 644 375
- US-A1- 2004 249 255

## Beschreibung

Die Erfindung betrifft ein mobiles Tonometer zur Durchführung einer berührungsfreien Selbsttonometrie und ein Verfahren zur automatischen Positionierung der Messachse eines solchen Tonometers.

Bekanntermaßen ist das Glaukom, auch Grüner Star genannt, eine der gefährlichsten Augenkrankheiten und einer der häufigsten Gründe für eine Erblindung in den Industrieländern. Eine der wichtigsten Ursachen der Krankheit ist ein erhöhter Augeninnendruck, auch bedingt durch Gefäßveränderungen. Bei zu hohem Druck kollabieren die Blutgefäße, wodurch die Nährstoffversorgung der Nerven unterbunden wird und ein Absterben derselben erfolgt. Die Schäden sind irreversibel und damit lebenslang. Sie äußern sich durch eine zunächst partielle Abnahme des Gesichtsfeldes und werden durch eine Kontrastangleichung in der Informationsverarbeitung und der unbewussten Nutzung von Erfahrungswerten zunächst nicht bemerkt. Erst ein extrem hoher Augeninnendruck äußerst sich in Kopfschmerzen und Druckgefühlen.

Zur Früherkennung eines Glaukoms wird meist eine Kombination aus Augeninnendruck-Messung (Tonometrie), Gesichtsfeldtest (Perimetrie), und einer Untersuchung des Sehnervenkopfes (Papillenbeurteilung) eingesetzt. Insbesondere bei der Beurteilung einer Druckmessung ist jedoch zu berücksichtigen, dass sich der Augeninnendruck im Verlaufe eines Tages und in Abhängigkeit vom körperlichen Zustand ähnlich dem Blutdruck ändert.

Bei gängigen Tonometern unterscheidet man Kontaktverfahren mit einer Berührung des Auges (z.B. Goldmann Applanationstonometrie) sowie kontaktlose bzw. berührungsfreie Messverfahren, auch "Non Contact Tonometrie" (NCT) genannt. Die "Non Contact Tonometrie" (NCT) bietet somit eine berührungs- und schmerzlose Möglichkeit der Früherkennung eines Glaukoms. Das Messprinzip der kontaktlosen Tonometrie basiert in herkömmlicher Weise auf einem linear oder rampenförmig steigenden Druckluft- oder Ultraschallimpuls, der die Hornhaut verformt, welche Verformung üblicherweise mittels IR-Strahlen gemessen wird. Da hierfür eine Anästhesie des Auges nicht notwendig ist, darf dieses Verfahren nicht nur von Ärzten, sondern grundsätzlich auch von Laien angewendet werden.

Bei der Früherkennung des Grünen Stars kommt der Tonometrie somit eine Schlüsselrolle zu, wobei der Augenarzt die verschiedenen Risikofaktoren zusammen mit der Messung des Augeninnendrucks bewertet. Da jedoch, wie bereits angesprochen, der Augeninnendruck aufgrund starker tageszeitlichen Schwankungen zum Zeitpunkt der Messung auch normal sein kann und Druckspitzen üblicherweise am frühen Morgen auftreten, werden diese den heutige üblicherweise beim Arzt durchzuführenden Untersuchungsmethoden nicht erkannt.

Die Einführung eines vom Probanden auf einfache Weise selbständig zu bedienenden Selbst-Non-Contact-Tonometer (SNCT), würde folglich einen Durchbruch bei der Glaukom-Diagnose und Therapie bringen, wobei das Tonometer auch von einem Laien ohne weitere Hilfestellung durch andere Personen-benutzt werden können und somit über einen vollautomatischen Messvorgang verfügen sollte. Ein Tonometer, das dem Probanden für die kostengünstige Messung eines Tagesprofils zur Verfügung gestellt wird und das einfach und ohne zweite Person zu bedienen ist, würde somit die Möglichkeiten der Früherkennung deutlich verbessern. Mit einem solchen Selbst-Tonometer könnten demnach tatsächlich aussagekräftige Daten erzeugt werden.

Veröffentlichungen zu Tonometern zur berührungsfreien Tonometrie sind vielfältig.

So betrifft die EP 1 310 209 A2 z.B. ein berührungsfreies Tonometer, bei welchem zur gezielten Krafteinwirkung auf das zu untersuchende Auge ein, über einen linear proportionalen Solenoid angetriebener Kolben Fluidpulse in vorbestimmter und kontrollierter Art und Weise generiert. Der Solenoid wird, basierend auf zuvor in einer digitalen Look-Up-Tabelle gespeicherten Daten, mittels eines Treiberstroms gesteuert. Ferner betrifft die EP 1 147 738 A1 ein Verfahren zur berührungsfreien Tonometrie, bei welcher während einer durch einen Fluidpuls bewirkten Korneadeformation mit bei konkavem Zustand der Applanation und mit bei konvexem Zustand der Applanation verbundene Beobachtungen verarbeitet werden, um den intra-okkularen Druckwert bereitzustellen. Die WO 00/02481 betrifft ein berührungsfreies Tonometer mit einem nicht linearen Druckanstieg während der Messung. Die WO 97/47232 betrifft ein berührungsfreies Tonometer mit einem bidirektionalen Linearmotor, der mit einem Kompressionsmechanismus antreibbar verbunden ist und auf ein Applanationssignal zur Reduzierung unerwünschter, auf das Auge übertragener Luftpulsenergie anspricht. Die für einen vollautomatischen Messvorgang notwendige Ausrichtung/Positionierung des Tonometers in Bezug auf das Auge sind jedoch nicht Gegenstand dieser Druckschriften, so dass die Lehren der offenbarten Tonometer auf ein auch für einen Laien bedienbares Selbsttonometer nicht anwendbar sind.

Die WO 99/51141 betrifft ein Applanations-Erfassungssystem für ein berührungsfreies Tonometer, welches eine Verringerung von Instrumentenausrichtungserfordernissen während des Messens ermöglicht. Ein durch einen Emitter ausgestrahltes und an der zu untersuchenden Kornea reflektiertes Licht wird durch ein Detektorfeld mittels einer Vielzahl von photosensitiven Detektoren erfasst und von diesen in eine Vielzahl von Lichtintensität repräsentierenden Signalkurven umgesetzt, worauf basierend eine optimale Signalkurve zur hierauf basierten Berechnung des intraokkularen Drucks bestimmt wird, so dass eine verbesserte Messauflösung bereitgestellt ist. Der zur Informationsverarbeitung benötigte technische Aufwand ist bei diesem Tonometer somit extrem groß und folglich kostenintensiv.

Die Druckschrift WO 95/20342 betrifft ein berührungsfreies, auf einem Tisch anzuordnendes Tonometer mit zusätzlicher pachymetrischer Messeinrichtung zur Dickenmessung der Kornea, wobei zur Ausrichtung/Positionierung auf der dem Probanden gegenüberliegenden Seite des Tonometers ein Bediener notwendig ist.

Die WO 03/039360 einschließlich zu der Familie gehörende weitere Druckschriften betreffen einen handgehaltenen berührungsfreien Tonometer, durch welchen ein Bediener das zu untersuchende Auge eines Probanden direkt entlang einer optischen Achse sehen kann, um mittels eines in dem Tonometer integrierten passiven Ausrichtungssystems das Tonometer auszurichten. Eine Bildanzeige wird hierzu mit dem Bild des gesehenen Auges überlagert, um dem Bediener eine Ausrichtung der drei Freiheitsgrade in Bezug auf die optische Achse zu ermöglichen. Eine Selbsttonometrie ist somit nicht möglich.

Die Offenlegungsschrift DE 196 47 114 A1 betrifft das berührungsfreie Messen des Augeninnendruckes, wobei die zur Verformung der Kornea notwendige Kraftwirkung unter Steuerung einer linearen oder quadratischen Zeitfunktion mittels eines Fluidstrahls oder durch Ultraschall und die Messung der Verformung mittels eines auf das Auge gerichteten Laserstrahls bewirkt wird. Der auf das Auge zu richtende Laserstrahl und die Richtung der Krafteinwirkung liegen auf einer Achse. Zumindest die Einrichtungen zur Richtung des Laserstrahls, zur Messung des rückreflektierten Laserstrahl und zur Krafteinwirkung sind starr mit einem Positionierungssystem verbunden und sind entweder auf einem zum Messobjekt in drei Koordinaten beweglichen Träger befestigt oder werden am Kopf des Probanden fixiert. Eine vom Probanden durchführbare Ausrichtung des Systems erfolgt ebenfalls unter Nutzung des, wenn auch abgeschwächten Laserstrahls, wobei ein Abgleich rückreflektierter Laserstrahlleistung erfolgt und dem Probanden zur Selbsttonometrie der Laser als Pilotlicht dient. Nach manuell durchgeführter und vom System durch Abgleich erkannter Ausrichtung erfolgt die Abstandseinstellung durch Projektion von im Zielgebiet zur Deckung zu bringenden Positionsmarken, durch Reflexion eines lateral einfallenden und auf einen Detektor reflektierenden Lichtbündels oder mittels eines Autofokussystems. Auch der Einsatz einer akustischen Ausrichtungskontrolle ist erwähnt, jedoch nicht weitergehend beschrieben. Im Falle einer Selbsttonometrie erfordert die Vorrichtung somit einen äußerst geübten Benutzer zur entsprechenden Ausrichtung.

Die Druckschrift DE 101 23 098 A1 betrifft ein Tonometer zur berührungsfreien Bestimmung des Augeninnendrucks, bei welcher Mittel zur Erfassung und Veränderung der relativen Lage des Tonometers gegenüber dem Auge und zur Erzeugung eines definierten Druckgasimpulses auf das Auge in Abhängigkeit von der relativen Lage vorgesehen sind. Die auch in Form eines Handtonometers vorgeschlagene Tonometeranordnung umfasst zur Ausrichtung und Lageerfassung drei um jeweils 2/3 π um die optische Achse gegeneinander versetzte Laserdioden mit fokussierbarem Kollimator und veränderbarer Blende zur Erzeugung von exakt parallelen Strahlengängen, die durch eine Abbildungsoptik auf das Auge gelenkt und von diesem durch die Abbildungsoptik auf einen, Transversalverschiebungen direkt anzeigenden und auf einem Träger angeordneten Sensor reflektiert werden. Die Abbildungsoptik und die Druckgasimpuls-Erzeugungsmittel sind koaxial zur optischen Achse der Tonometeranordnung angeordnet. Der nach Erfassen einer definierten Lage von Hand oder automatisch ausgelöste Druckgasimpuls auf das Auge bewirkt wiederum eine Veränderung der Lage und somit eine Auslenkung der reflektierten und zuvor zur Ausrichtung eingesetzten Strahlengänge, die zur Auswirkung des Druckgasimpulses durch das gleiche, zuvor zur Bestimmung der exakten Positionierung eingesetzten Sensorsystem entsprechend ausgewertet werden. Auch diese Anordnung erfordert somit im Falle eines Handtonometers einen äußerst geübten Benutzer, da lediglich passive Mittel zur Lageerfassung bereitgestellt sind.

Die EP 1121 895 B1 einschließlich zu der Familie gehörende weitere Druckschriften betreffen ein handgehaltenes berührungsfreies Tonometer, wobei eine Positionierungseinrichtung derart vorgesehen ist, dass ein Positionierungsbild dem Probanden zur Selbstpositionierung bereitgestellt wird. Ein auf das Auge gerichteter und von der Kornea reflektierter Infrarotstrahl wird bei korrekter Positionierung durch den Probanden senkrecht von einer Messachse auf einen Detektor zur passiven Monitorausrichtung derart abgelenkt, dass ein Triggersignal zum Auslösen des Messvorgangs aktiviert wird. Die aktive Positionierung erfolgt somit wiederum durch den Probanden, welches somit gleichermaßen einen äußerst geübten Benutzer bedarf.

Ein Verfahren gemäß der Präambel von Anspruch 1 ist aus des WO 98/09564 bekannt.

Aufgabe der Erfindung ist daher ein flexibel einsetzbares Tonometer zur Durchführung einer berührungsfreien Selbsttonometrie, ein sogenanntes Self-Non-Contact-Tonometer (SNCT) sowie ein aktives Positionieren für ein solches bereitzustellen, welches auch von ungeübten Probanden auf einfachste Weise und ohne weitere Hilfe zur korrekten Augeninnendruckmessung selbstbedienbar einsetzbar ist und folglich ferner die kostengünstige Aufnahme auch von Tagesdruckprofilen, also die mehrfache Messung des Augeninnendrucks zu verschiedenen Zeitpunkten sicherstellt.

Die erfindungsgemäße Lösung der Aufgabe ist auf höchst überraschende Weise bereits durch einen Gegenstand mit den Merkmalen nach einem der anhängigen unabhängigen Ansprüche gegeben.

Vorteilhafte und/oder bevorzugte Ausführungsformen und Weiterbildungen sind Gegenstand der Unteransprüche.

Wesentliche Vorteile der Erfindung bestehen somit darin, dass aufgrund des aktiven Positionierungssystems bzw. der automatisierten Aktivpositionierung der Einfluss eines Probanden auf die Messunsicherheit bei SNCTs durch ungenaue manuelle Positionierung und die bisher notwendigen Handlungen des Probanden deutlich reduziert wird und folglich auch bei ungeübten Probanden die Handhabung und Tonometerbedienung auf ein Minimum begrenzbar ist.

Die vordefinierte/vordefinierbare Justierung und/oder Nachführung der aktiven Positionierung umfasst zweckmäßigerweise ferner die Positionierung der Messachse in einem definierten Abstand zur Hornhaut, so dass die Messachse bevorzugt in zumindest drei Freiheitsgraden einstellbar ist.

Aufgrund der unter Ansprechen auf die automatisch durchgeführte Detektion der Blickrichtung automatisch vom System positionierten, also justierten bzw. nachgeführten Messachse, entfällt darüber hinaus die bisherige Ausrichtung der optischen Achse auf eine vordefinierte Messachse durch den Probanden, insbesondere da die Messachse des erfindungsgemäßen Gerätes und die optische Achse des Auges zur Durchführung der Messung nicht mehr zwingend in einer fest vordefinierten Relation zueinander ausgerichtet sein müssen.

Vorzugsweise wird das Positionierungssystem durch eine Steuer-/Regeleinheit derart überwacht, dass die Detektion kontinuierlich oder periodisch durchgeführt wird und die Messachse entsprechend nachgeführt wird. Somit können mögliche Änderungen der Blickrichtung, insbesondere unmittelbar vor einer Messung oder zwischen einzelnen zeitlich beabstandet aufeinanderfolgenden Messungen, kompensiert werden.

Für eine effektive Reduzierung der notwendigen Sensorik und Rechnerleistungen werden für die erfindungsgemäße Lagendetektion zur Detektion der Blickrichtung eingesetzte Signale bevorzugt analog verarbeitet.

Hierzu umfasst die Detektionseinrichtung vorzugsweise Mittel zur Erfassung von geometrischen Abmessungen der Hornhaut, wobei die Mittel bevorzugt zur Nutzung von optischen Strahlen, d.h. zur Nutzung von sichtbaren Strahlen oder insbesondere zur Nutzung von IR-Strahlen, ausgebildet sind.

Gemäß bevorzugter Ausführungsformen umfasst das Tonometer wenigstens einen optischen Sender, z.B. einen IR-Sender, zum gerichteten Senden eines Strahls wenigstens teilweise auf die Hornhaut und wenigstens einen optischen Empfänger, z.B. einen IR-Empfänger, zum Empfangen wenigstens eines Strahlenanteils des gerichteten Strahls und/oder eines reflektieren Strahls. Da folglich zur Detektierung von geometrischen Hornhautabmessungen Relativ-, Absolutsignale und/oder Abschattungseffekte bei jeweiligen Strahlengängen gemessen werden können, ist die jeweilige Sensorik Tonometerspezifisch anpassbar und einsetzbar.

Die Nutzung von geometrischen Abmessungen, also einschließlich auch von im Wesentlichen rotationssymmetrischen Geometrien, für eine analoge Blickrichtungserfassung im Nahfeld gewährleistet folglich auf einfache Weise eine zuverlässige Bestimmung auch des Hornhautscheitels und folglich der Lage einer im Wesentlichen jeden Stelle auf der Hornhautoberfläche, insbesondere im mittleren oder in einem im Wesentlichen zentralen Bereich der Hornhaut, zur Ausrichtung der Messsystems.

In Weiterbildung ist vorgesehen, dass das Tonometer wenigstens eine Reflektoreinrichtung umfasst, die mit jeweils einem optischen Sender, insbesondere IR-Sender, und einem optischen Empfänger, insbesondere IR-Empfänger, zur Bildung eines geometrische Hornhautabmessungen markierenden Strahlengangs zusammenwirken.

Sind zwei optische Sender, insbesondere IR-Sender, und optische Empfänger, insbesondere IR-Empfänger, mit jeweils zugeordneter Reflektoreinrichtung zur Bildung eines, geometrische Hornhautabmessungen markierenden Strahlengangs vorgesehen, kann auf einfache Weise zur Bestimmung von Lagen in den Freiheitsgraden des Auges und also der Blickrichtung dieses von zwei parallelen Strahlengängen käfigartig umstrahlt werden.

In ergänzender oder alternativer Ausbildung ist ferner vorgeschlagen, wenigstens einen, insbesondere als IR-Sender ausgebildeten Sender und einen, insbesondere als IR-Empfänger ausgebildeten Empfänger vorzusehen, die für eine auf der Reflexion eines Strahlengangs basierende Auswertung von Lagen in den Freiheitsgraden entsprechend positionierbar sind. Hierdurch ist ein Verfahren gewährleistbar, bei welchem die Reflexion eines Strahlenganges in einem Absolutsignal ausgewertet wird.

In weiterer ergänzender oder alternativer Ausbildung ist ferner vorgeschlagen, die Reflexion eines Lichtkegels von wenigstens einem optischen Sender an der Hornhautoberfläche von einer Mehrzahl um den Sender angeordneten, z.B. vier rotationssymmetrisch um den Sender angeordneten, optischen Empfängern zu erfassen und basierend auf der auswertenden Verarbeitung von Relativ- und Absolutsignalen, also im Wesentlichen von relativen Verhältnissen und absoluten Pegeln empfangener Strahlenganganteilen bzw. Strahlungsflüsse, die Lage einer Stelle an der Hornhautoberfläche zu bestimmen.

In bevorzugten Ausführungen umfasst das Positionierungssystem somit von der Steuer-/Regeleinheit kontrollierbare Führungen zum Justieren des wenigstens eines optischen Senders, des wenigstens eines optischen Empfängers und/oder der wenigstens einen Reflektoreinrichtung umfasst.

Entsprechend sind auch der zu justierenden bzw. nachzuführenden Messeinheit mittels der Steuer-/Regeleinheit kontrollierbare Führungen zugeordnet.

Derartige Führungen sind zweckmäßigerweise zur Positionierung von Sensor- und/oder Mess(teil)einheiten in im Wesentlichen drei senkrecht zueinander verlaufenden Freiheitsgraden ausgebildet.

Die Steuer-/Regeleinheit zur erfindungsgemäßen Aktivpositionierung sowie eine Datenauswerte- und Datenspeichereinheit können im Tonometer integriert sein und/oder das Tonometer kann eine mit entsprechenden, jedoch entfernten Einheiten korrespondierende Sende- und/oder Empfangseinrichtung umfassen.

Für ein möglichst geringes Führungsspiel, zur Verschleißminimierung und folglich zur Erhöhung der Wartungsfreiheit, ist ferner vorgesehen, Führungen zum Bewegen von justierbar angeordneten (Teil-)Einrichtungen des Positionierungssystems und des Messsystems mit Festkörpergelenken auszubilden, insbesondere mit Festkörpergelenken in Art von Filmscharnieren und/oder Federführungen.

Um ein optimales Wirkungsverhältnis zu erreichen, ist ferner die Nutzung von Kinematiken, d.h. Lager und entsprechende Verbindungen zwischen einzelnen Lagern umfassende Führungen, vorgesehen, die zum Reduzieren des Montageaufwandes z.B. jeweils monolithisch, insbesondere durch Spritzguss-, Galvanik- oder Lasertechnik, hergestellt sind.

Die Festkörpergelenkführungen und/oder Kinematiken sind ferner von, von der Steuer-/Regeleinheit kontrollierbaren, Aktoren betrieben.

Für eine einfache technische Umsetzung eignen sich bevorzugt translatorische und/oder rotatorische Führungskinematiken, da zum Betreiben derartiger Kinematiken applikationsspezifisch auf eine Vielzahl von unterschiedlichen Aktoren zurückgegriffen werden kann. Derartige Aktoren können somit bevorzugt auf Drehspulsystemen, Schrittmotoren, Linearantrieben oder Tauchspulen basieren.

In einer zweckmäßigen Weiterbildung ist vorgesehen, einen im wesentlichen vertikal verlaufenden translatorischen Freiheitsgrad des Positionierungs- und/oder Messsystems durch eine Rotation anzunähern, so dass das Eigengewicht in den rotatorischen Lagern getragen wird, welches folglich zu einer wesentlich verbesserten Stabilisierung des Justiervorgangs beiträgt. Auch ist hierdurch der Bedarf an benötigtem Bauraum im Vergleich zu einer einsetzbaren translatorischen Führung reduzierbar. Liegt ferner der Drehpunkt der Rotation im Schwerpunkt der zu justierenden (Teil-)Einrichtung, ist folglich nur ein Minimum an notwendigem Energieeinsatz erforderlich.

Gemäß bevorzugter Ausführungsformen ist das erfindungsgemäße Tonometer Brillen-artig oder Helm-artig ausgebildet oder ein mit der Hand zu haltendes Handtonometer.

Insbesondere im Fall eines Handtonometers sind für eine stabile Grundpositionierung bevorzugt Anlagenbereiche vorgesehen, die zum Ansetzen an ein Nasenbein, eine Schläfe und/oder einen Wangenknochen entsprechend geformte und/oder formbare Stützbereiche umfassen, wobei die Anlagenbereiche in deren Position vorzugsweise einstellbar fixierbar sind.

Um die automatisierten Ausrichtmechanismen weiter zu vereinfachen, ist ferner, insbesondere im Falle des Handtonometers vorgeschlagen, dass das Tonometer zusätzlich eine Peileinrichtung mit einem dem Probanden zugewandten Tubus zur verbesserten Grundpositionierung der optischen Achse des Auges umfasst.

Bei einer bevorzugten, technisch sehr einfachen, jedoch bereits sehr effektiven Ausführungsform umfasst die Peileinrichtung ein mit dem Tubus koaxial angeordnetes optisches Peilmuster, welches bei axialen Versatz gegenüber der optischen Achse wenigstens teilweise verdeckt bzw. nicht vollständig sichtbar ist.

Eine weiterverbesserte Stabilisierung der Grundposition wird im Falle des Handtonometers gewährleistet, wenn das Positionierungssystem und Messsystem in einem Gehäuse des Tonometers angeordnet sind, mit welchem schwingungsentkoppelt wenigstens ein Haltegriff verbunden ist. Insbesondere unter Nutzung eines passives Feder-Dämpfer-Systems kann Schwankungen oder einem Zittern des Probanden effektiv entgegengewirkt werden. Eine solche Schwingungsentkopplung ist somit grundsätzlich auch für Tonometer mit gegenüber den unabhängigen Ansprüchen anderen Merkmalen in äußerst zweckmäßiger Weise einsetzbar.

Um einen definierten, insbesondere linearen oder nicht linearen, Druckanstieg während der Messung zu gewährleisten, umfasst das Tonometer gemäß der Erfindung bevorzugt ferner eine, zweckmäßiger Weise zeitbasiert steuerbare Druckbeaufschlagungseinrichtung zum definierten Zuführen von Druck, insbesondere basierend auf Luft, auf das Auge, die z.B. auch zwischen einer originären Druckerzeugungseinrichtung und einem Düsenkopf angeordnet ist.

Ergänzend oder alternativ ist ferner vorgesehen, das Tonometer mit einer Druckbeaufschlagungseinrichtung, insbesondere einen Luftstoßgenerator umfassend, auszubilden, welche einen austauschbaren Düsenkopf aufnimmt. Hierdurch ist auf äußerst wirksame Weise ein Infektionsschutz, insbesondere gegen HIV und Herpes-Viren aus Tränenfilmtröpfchen, gegeben, da der Düsenkopf für jede Messung oder bei Einsatz des Tonometers durch einen neuen Probanden ausgetauscht werden kann und eine zeitintensive Reinigung somit grundsätzlich entfällt. Von Vorteil ist ferner, dass ein solcher austauschbarer Düsenkopf grundsätzlich auch für Tonometer mit gegenüber den unabhängigen Ansprüchen anderen Merkmalen in äußerst zweckmäßiger Weise einsetzbar ist.

Umfasst der austauschbare Düsenkopf eine die Düsenöffnung in radialer Richtung zur Zuführrichtung umgebende Schirmung, ist sowohl der funktionale Teil der Düse wie auch deren Umgebung infektionsgeschützt bzw. austauschbar. Bei zumindest teilweise transparenter Ausbildung der Schirmung, ist diese auch für eine Anordnung unmittelbar vor der Messsensorik ausbildbar.

Um die Druckbeaufschlagung gezielt auf das Auge zu leiten, umfasst die Druckbeaufschlagungseinrichtung ferner bevorzugt eine beweglich angeordnete Einrichtung, welche den Düsenkopf zum Einleiten des Druckbeaufschlagungsmediums aufnimmt, wobei die bewegliche Einrichtung zweckmäßigerweise mit der Ausrichteinrichtung gekoppelt ist.

Weitere Vorteile und Merkmale der Erfindung werden aus der nachfolgenden beispielhaften Beschreibung bevorzugter Ausführungsformen unter Bezugnahme auf die beigefügten Zeichnungen ersichtlich. In den Zeichnungen zeigen:
- Fig. 1a: schematisch eine Blickrichtungserfassung durch Nutzung der Abschattung eines Strahlenganges,
- Fig. 1b: schematisch eine Blickrichtungserfassung durch Nutzung eines "Käfig" aus zwei parallelen Strahlengängen,
- Fig. 1c: schematisch eine Blickrichtungserfassung durch Nutzung der Auswertung des Absolutsignals eines reflektierten Strahlungsflusses,
- Fig. 1d: schematisch eine Blickrichtungserfassung mittels im Wesentlichen lediglich einer Nutzung der Rotationssymmetrie des Auges zur Lageerfassung einer Stelle auf der Hornhaut unter Einsatz eines Emitters und vier rotationssymmetrisch um den Emitter angeordneten Photodioden,Fig. 2a die Grundpositionierung eines Handtonometers gemäß der Erfindung vor dem Auges basierend auf drei Anlagebereichen,
- Fig. 2b: stark vereinfacht drei bevorzugte Stützstellen als Anlagebereiche des Handtonometers zum Ansetzen an den Schädel eines Probanden nach Fig. 2a, die eine reproduzierbare Ausgangsposition für die anschließende Feinpositionierung und Messung bieten,
- Fig. 3a: eine Brillenähnliche Konstruktion des Tonometers,
- Fig. 3b: eine Helmähnliche Konstruktion des Tonometers,
- Fig. 4: eine Messachse im Lot der Hornhaut, die bei der dargestellten Ausführung gegenüber der optischen Achse verdreht ist,
- Fig. 5a u. b: schematisch die Wirkung einer integrierten Peileinrichtung gemäß der Erfindung, wobei konzentrische Ringe eines Positionslichtes, die sich nur bei einer korrekten Positionierung symmetrisch darstellen (Fig. 5a), bei einem axialen Versatz ein verschobenes Muster darstellen (Fig. 5b),
- Fig. 6: stark schematisiert in teils aufgebrochener Ansicht ein beispielhaftes Kinematikprinzip zum Aktor-betriebenen translatorischen und rotatorischen Bewegen von Sensor- und Mess(teil)einheiten, und
- Fig. 7: schematisch ein Disposable umfassend eine Düse mit ausgeführtem Spritzschutz.

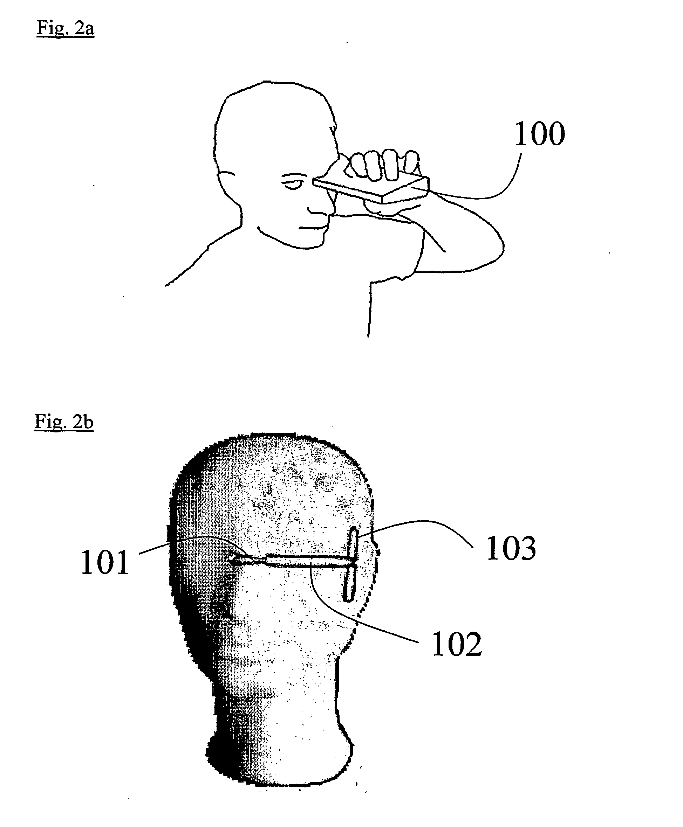

Nachfolgend wird zunächst auf Fig. 2a und 2b Bezug genommen. Fig. 2a zeigt stark vereinfacht ein erfindungsgemäßes mobiles Tonometer in Art eines Handtonometers 100, welches zur Grundpositionierung für die Selbstdurchführung einer berührungsfreien Tonometrie, nachfolgend als SNCT bezeichnet, mit drei Anlagebereichen ausgebildet ist, so dass das Handtonometer für die eigentliche Messung am Schädel des Probanden durch diesen selbst auf einfache Weise an drei vordefinierten Stützstellen ansetzbar ist.

Die Anlagebereiche umfassen in bevorzugter Ausführung derart formbares Material oder sind derart beweglich mit dem Tonometer verbunden, dass zum Einen eine gewisse individuelle Anpassung an unterschiedliche Gesichtskonturen und zum Anderen eine einstellbare Fixierung zur Gewährleistung einer reproduzierbaren Ausgangsposition, bewirkt werden kann.

Im vorliegenden Beispiel sind die Anlagebereiche, wie bei Fig. 2b angezeigt, zur Anlage an das Nasenbein 101, an die Schläfe 102 und die Wangenknochen 103 entsprechend ausgebildet. Anlagenbereiche mit entsprechend ausgebildeten Stützstellen sind somit für eine stabile Grundposition des Handtonometers besonders zweckmäßig.

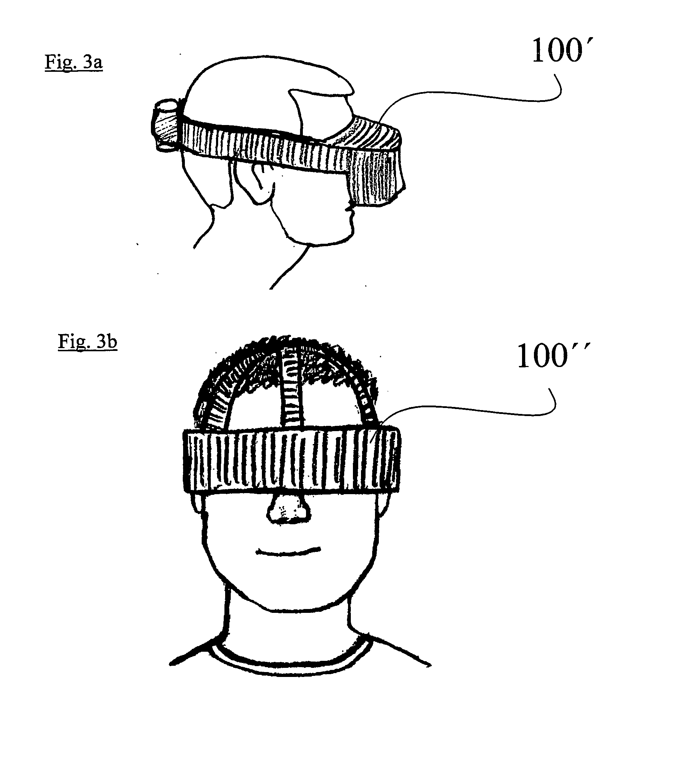

Die Fig. 3a und die Fig. 3b zeigen weitere Ausführungsformen eines erfindungsgemäßen mobilen Tonometers in Art einer Brille 100' bzw. in Art eines Helms 100". Derartige Ausführungen sind folglich mit einer Vielzahl von zusätzlichen und/oder anderen Stützstellen umfassenden Anlagebereichen, verglichen mit einem von Hand gehaltenen Tonometer, ausbildbar, um eine individuell, flexibel einstellbare und reproduzierbare Grundpositionierung für den Probanden auf einfachste Weise zu ermöglichen.

Ferner umfasst ein erfindungsgemäß bevorzugtes Tonometer neben einem integrierten Messsystem zur Durchführung der berührungsfreien Tonometrie ein integriertes aktives, durch Aktoren betriebenes Positionierungssystem zur Positionierung des Messsystem in Bezug auf ein zu untersuchendes Auge, welches durch wenigstens eine Steuer-/Regeleinheit kontrolliert wird. Die wenigstens eine Steuer-/Regeleinheit kann hierbei im Tonometer integriert sein oder entfernt sein, wobei in diesem Fall das Tonometer eine Sende- und/oder Empfangseinrichtung zur entsprechenden Datenübertragung umfasst. Die verbleibende relative Ausrichtung des Auges zur Messeinheit des Handtonometers wird somit erfindungsgemäß automatisch von der nachfolgend beschriebenen optischen Sensorik erfasst und mittels des Fein-Positioniersystems kompensiert, so dass in Folge die Messeinheit des erfindungsgemäßen Tonometers vollautomatisch im Lot der Hornhaut, bei einigen Ausführungsformen vollautomatisch im Lot des Hornhautscheitels positioniert wird.

Da somit durch den Probanden die Position des Handtonometers am Kopf mittels der stützend ansetzbaren Anlagebereiche nur grob vorgegeben werden muss, ist ferner der Einfluss des Probanden auf die Messunsicherheit bei der SNCT durch ungenaue manuelle Positionierung deutlich reduziert und lediglich eine minimale Handhabung und Gerätebedienung durch den Probanden erforderlich.

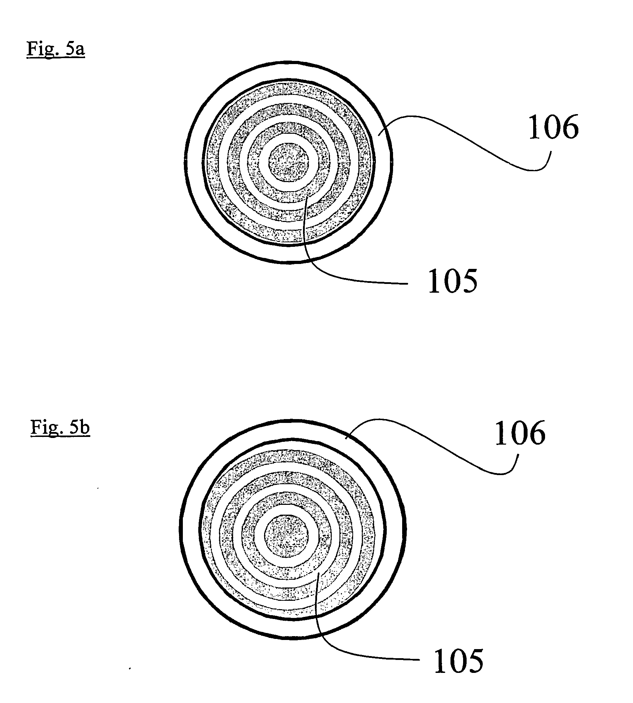

In besonders zweckmäßiger Weiterbildung ist jedoch zusätzlich für die Sensorik und das Fein-Positionierungssystem unterstützend vorgesehen, das Tonometer mit einer optischen Peileinrichtung und mit einem Tubus für den Probanden zur ergänzenden Grundausrichtung der optischen Achse des Auges auszubilden. Hierdurch wird dem Probanden zumindest vor dem Auslösen der automatischen Feinpositionierung und anschließenden Messung des Augeninnendrucks ein Positionslicht angezeigt, mit dessen Hilfe er das SNCT bzw. dessen Messeinheit im Wesentlichen zentral vor einem Auge vorpositionieren kann. Wie bei Fig. 5a und Fig. 5b schematisch dargestellt, umfasst ein solches Positionslicht z.B. ein aus konzentrischen Ringen bestehendes optisches Peilmuster 105, welche grundsätzlich koaxial zu einem Tubus 106 angeordnet sind. Blickt der Proband durch den Tubus 106, ist das Peilmuster 105 bei axialem Versatz der optischen Achse des Auges teilweise verdeckt, wie bei Fig. 5b zu sehen, und bei ebenfalls koaxialer Ausrichtung vollständig für den Probanden sichtbar (Fig. 5a).

Da bei entsprechender Vorausrichtung die mittels Aktorsteuerung abzudeckenden Winkel- und Wegpositionierungen für die Sensorik und das Fein-Positionierungssystem deutlich reduziert werden können, sind in Folge auch die äußeren Abmessungen des Tonometers aufgrund der für die erfindungsgemäß automatisch bewirkte Blickrichtungserfassung benötigten reduzierten Bewegungsfreiheiten effektiv zu begrenzen, welches insbesondere im Falle eines Handtonometers wiederum dem Probanden zur weiter vereinfachten Handhabung bei der Durchführung einer berührungsfreien Selbsttonometrie verhilft.

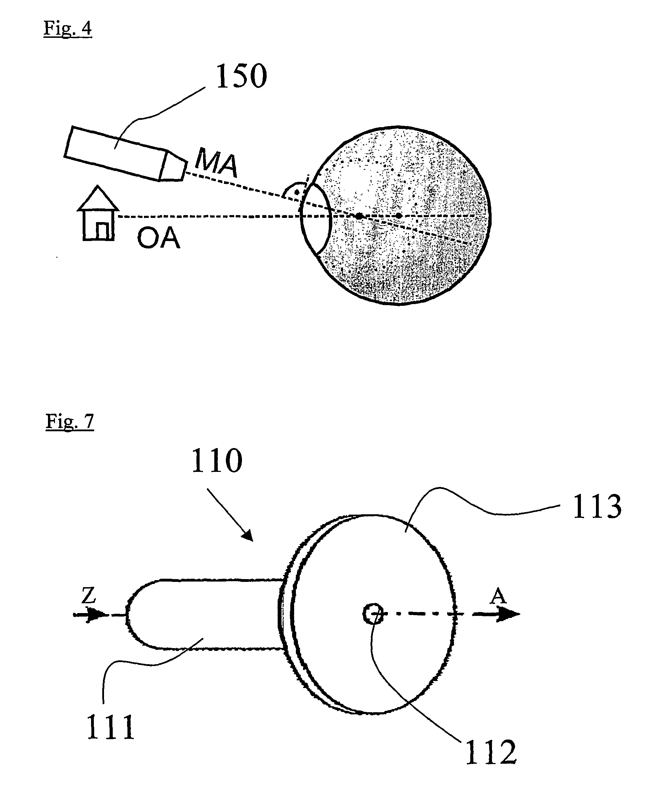

Diese aktive Positionierung des Messsystems des Selbsttonometers in drei Dimensionen gemäß der Erfindung stellt im Wesentlichen die automatische Positionierung der Messwerte aufnehmenden Messeinheit 150, und damit der Kerneinheit des Messverfahrens, vor dem Auge zweckmäßiger Weise derart bereit, dass die Messachse MA im Lot der Hornhaut, wie z.B. bei Fig. 4 dargestellt, ausgerichtet ist, wobei im vorliegenden Beispiel eine Düse zur Druckbeaufschlagung des zu untersuchenden Auges in Richtung der Messachse MA in der Messeinheit 150 aufgenommen ist. Es sei jedoch darauf hingewiesen, dass Düse und Messeinheit auch separat, insbesondere parallel zueinander, anordenbar sind und durch jeweils zugeordnete Führungen an der Messachse ausrichtbar sind. Von besonderem Vorteil ist somit, dass die Messachse MA nicht mehr starr an die mit der nachfolgend erfindungsgemäß erfassten Blickrichtung in Zusammenhang stehende optische Achse OA des Auges gekoppelt ist, sondern, wie bei Fig. 4 zu sehen, auch gegenüber der optischen Achse OA verdreht sein kann.
Auch wenn beide Achsen z.B. bei geforderter optimaler bzw. noch präziserer Ausrichtung auf den Hornhautscheitel zusammenfallen können, muss das erfindungsgemäße Tonometer weder zwingend in der optischen Achse OA des Auges messen, noch müssen die Messachse MA und die optische Achse OA des Auges zwingend in einer fest vordefinierten Relation zueinander ausgerichtet werden.

Hierdurch ist eine äußerst vielseitige und flexible Anwendung des erfindungsgemäßen Tonometers auch bei unterschiedlichsten Augenrandbedingungen gewährleistet.

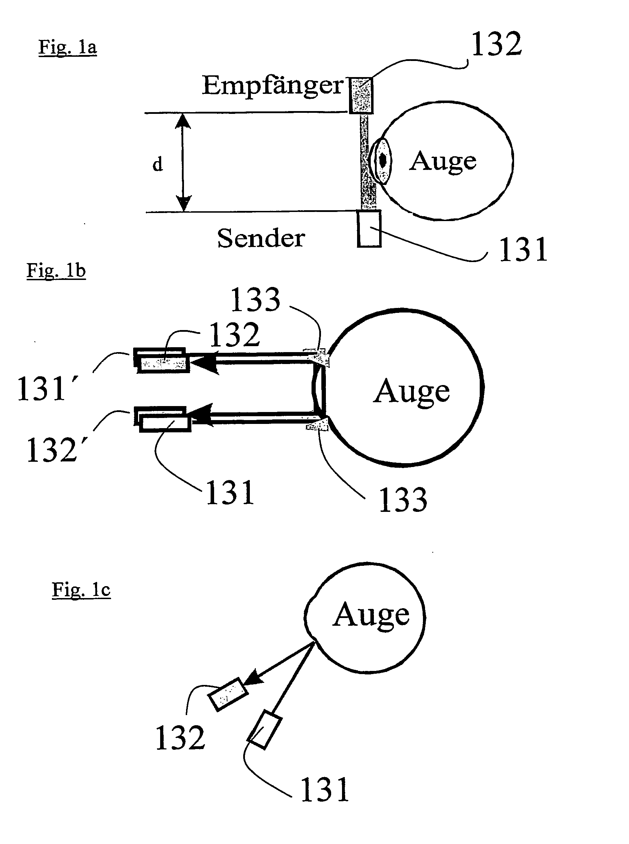

Unter nachfolgender Bezugnahme auf die Fig. 1a, 1b und 1c wird eine beim erfindungsgemäßen Tonometer bevorzugt eingesetzte optische Sensorik zur Blickrichtungserfassung beispielhaft beschrieben. Insbesondere, um den Sensor- und Rechenaufwand zur Blickrichtungserfassung, ggf. mit entsprechender Detektierung der optischen Achse, als auch der nachfolgend zu justierenden Messachse auf ein Minimum zu begrenzen, werden vorzugsweise im Wesentlichen nur geometrische Abmessungen der Hornhaut für eine analoge Blickrichtungserfassung im Nahfeld genutzt.

Für das analoge Erfassen einer solchen Augengeometrie ist eine Anzahl von unterschiedlichsten Sensorischen Detektionstechniken nutzbar, wobei die Erfindung den Einsatz von optischen Strahlen, bevorzugt den Einsatz von IR-Strahlen, unter Anwendung folgender Detektionstechniken vorschlägt.

Der Sensorikeinheit zugeordnet ist eine nicht näher dargestellte und beschriebene Auswerteeinheit, welche die hierbei ermittelten (Zwischen-)Daten auswertet. Die Auswerteeinheit kann im Tonometer integriert sein oder ausführungsabhängig als separate, mit dem Tonometer über entsprechende kabelgebundene oder -lose Sende-/Empfangseinrichtung in Verbindung stehende Auswerteeinheit verwirklicht sein.

Bei der Funktionsskizze gemäß Fig. 1a ist schematisch die Nutzung der Abschattung eines Strahls oder mehrerer Strahlen dargestellt. Ein Sender 131 und ein Empfänger 132 sind hierzu in vordefiniertem oder auch einstellbaren Abstand "d", z.B. von ca. 40mm, derart zueinander positioniert, dass bei ungestörtem Strahlengang zwischen Sender 131 und Empfänger 132 ein vom Sender 131 ausgehender Strahl verlustfrei vom Empfänger 132 erfasst wird. Unter Beibehaltung dieser relativen Positionierung des Senders 131 und des Empfängers 132 zueinander, sind oder werden diese in der Nähe des Auges derart angeordnet, dass der Pfad oder auch die Ebene eines Strahlengangs durch die Hornhaut teilweise abgeschattet wird. Zur automatischen Bestimmung der Lage, bspw. der Position und Ausrichtung, der Hornhaut, werden somit jeweils mittels der Sensorikeinheit detektierte, nicht, teilweise oder ganz abgeschatteten Strahlengänge ausgewertet und auf der Auswertung und/oder Anzahl der ausgewerteten Strahlengänge basierend ein Regelsignal erzeugt, zum Justieren und Nachführen der Messeinheit 150 und Düse im Lot bspw. des Hornhautscheitels. Die Sensorikeinheit mit Sender 131 und Empfänger 132 ist bevorzugt Aktor-betrieben, so dass basierend auf der Strahlengangauswertung auch ein Nachführen der Sensorikeinheit selbst in Bezug auf den Hornhautscheitel gewährleistet ist. Aufgrund von an sich bekannten geometrischen Abmessungen von Auge und Hornhaut sind für eine bewusste Positionierung im Lot des Hornhautscheitels folglich üblicherweise bereits vier Stützstellen ausreichend. Eine geforderte Positionierung lediglich im mittleren Bereich der Hornhaut führt somit zu einer nochmals weitergehenden Reduzierung des Sensor- und Rechenaufwands.

Bei der Funktionsskizze gemäß Fig. 1b ist schematisch die Bildung eines "Käfigs" aus wenigstens zwei parallelen Strahlengängen dargestellt, mit welchem auf einfache Weise bereits Lagen in zwei Freiheitsgraden des Auges bestimmbar sind. Hierzu umfasst die optische Sensoreinheit zu jeweils einem Sender/Empfängerpaar 131 und 132 bzw. 131' und 132' zugeordnete Reflektoren 133, die in einer, die Hornhaut umfassenden Ebene angeordnet sind bzw. werden. Auch bei einer solchen Anordnung ist die Sensorikeinheit insgesamt oder sind die einzelnen Teileinheiten individuell über Aktoren zweckmäßig bewegbar, um eine Anpassung an jeweilige Augenrandbedingungen zu gewährleisten und/oder verschiedene Positionen zur Blickrichtungserfassung einzunehmen. Auch bei dieser Art von Detektionstechnik werden somit Absolutsignale als auch durch Abschattung der Strahlengänge durch die Hornhaut hervorgerufene Relativsignale zur im Wesentlichen exakten Lagebestimmung der Hornhaut genutzt.

In weiterer ergänzender oder alternativer Ausführung zu der bei Fig. 1b dargestellten Anordnung, wird gemäß Fig. 1c die Positionierung der Sensorikeinheit geregelt, wobei die Reflexion eines Strahlungsgangs im Absolutsignal somit z.B. zur Lagenbestimmung innerhalb eines weiteren Freiheitsgrades ausgewertet wird. Hierzu sind der Sender 131 und der Empfänger 132 vorzugsweise als individuell bewegliche Sensorik-Teileinheiten ausgebildet, so dass die Auswerteeinheit anhand der mittels der Aktorik jeweils angesteuerten Positionen des Senders 131 und des Empfängers 132 und der durch die Hornhaut bewirkten und in Folge detektierten Reflexion des Strahlenganges die entsprechende Auswertung der Blickrichtung vornimmt. Auch die Auswertung in allen drei Freiheitsgraden aus der Reflexion eines Strahlungsgangs im Absolutsignal ist somit gegeben.

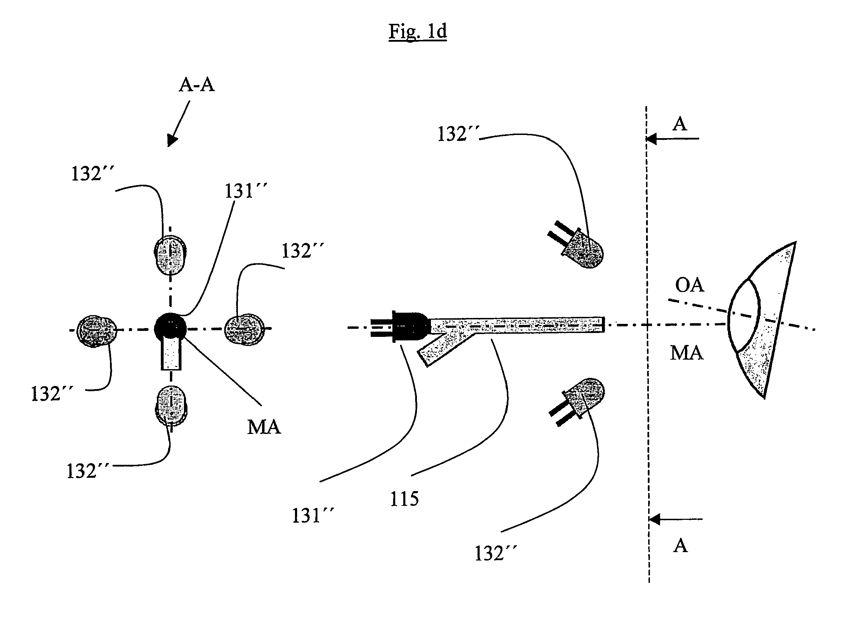

Fig. 1d zeigt skizzenhaft (in Schnittansicht entlang der Messachse MA und aus Sicht des Auges entlang der Linie A-A) eine weitere ergänzende oder alternative Ausführungsform zur Bestimmung einer Hornhautlage zur Positionierung einer Messeinheit im Lot. Im Wesentlichen lediglich unter Ausnutzung der rotationssymmetrischen Eigenschaften der geometrischen Abmessungen der Hornhaut wird die Reflexion eines Lichtkegels eines Emitters 131" an der Hornhautoberfläche von um den Emitter 131" herum angeordneten optischen Empfängern 132", beim dargestellten Beispiel von vier rotationssymmetrisch angeordneten Photodioden 132", erfasst. Der Emitter 131" ist hierzu z.B. zentriert am Ende einer Düse 115 zum Aufbringen eines Luftstoßes auf das Auge angebracht. Durch Auswertung der absoluten Pegel und zweckmäßigerweise der relativen Verhältnisse der jeweils empfangenen Strahlungsflussanteile kann die Lage einer Hornhautoberflächenstelle ermittelt werden, an welcher die Messachse MA der dargestellten Messeinheit nachfolgend im Lot aktiv positioniert wird. Bei der Ausführungsform gemäß Fig. 1d wird direkt der emittierte und reflektierte Lichtkegel des Emitters 131" auf die Empfangselemente 132" abgebildet, wobei in vorteilhafter Weise nur ein Bauelement, der Emitter 131', präzise ausgerichtet werden muss. Der Einsatz von einfachen Photodioden 132" als Empfangselemente erhöht zusätzlich den wirtschaftlichen Nutzen.

Je nach Ausführungsform der jeweils eingesetzten Sensorik werden folglich Absolutsignale und/oder Relativsignale zur Lagebestimmung, also insbesondere der Position oder der Position und Ausrichtung bzw. Orientierung, der Hornhaut erfasst und ausgewertet und in funktioneller Abhängigkeit die Bestimmung eines Lots bspw. des Hornhautscheitellots vorgenommen. Ein Relativsignal basiert somit im Allgemeinen auf einem relativen Verhältnis von mehreren Strahlenflüssen oder verarbeiteten Messgrößen, wohingegen ein Absolutsignal im Wesentlichen auf dem absoluten Pegel eines Strahlenflusses oder einer verarbeiteten Messgröße basiert.

Die Auswertung erfolgt bevorzugt on-line, so dass hierüber ferner eine entsprechende Neupositionierung und/oder Nachführung der Aktor-betriebenen Sensorikeinheit oder Sensorikteileinheiten regelbar ist, um die Lage der Hornhaut in bestimmten Abständen, insbesondere unmittelbar vor einer Messung oder zwischen zeitlich kurz beabstandeten Messungen, zu verifizieren und darauf basierend eine im Lot der Hornhaut bspw. im Lot des Hornhautscheitels auszurichtende Messachse MA zu bestimmen, in einem definierten Abstand der Messeinheit zur Hornhaut zu positionieren, kontinuierlich zu kontrollieren und gegebenenfalls nachzujustieren.

Nach erfolgter Feinpositionierung der Messeinheit und Düse folgt der eigentliche Messvorgang des Augeninnendrucks, bei dem ein auf die Hornhaut gerichteter Druckimpuls, insbesondere ein Luftdruckimpuls, die Hornhaut leicht deformiert und aus dem Bewegungsverhalten der Hornhaut der vorliegende Augeninnendruck berechnet wird.

Der exakte Messwert wird z.B. in einem Speicher des Tonometers abgelegt, den der behandelnde Augenarzt bei der nächsten Untersuchung ausliest. In alternativer Ausführung kann mittels Sende-/Empfangseinrichtung des Tonometers auch die Speicherung der Messwerte in einem entfernten Speicher, auf welchen der Arzt Zugriff hat, erfolgen. Zweckmäßiger Weise erhält der Proband ferner selbst nach jeder einzelnen Messung eine Rückmeldung, z.B. in der Form, dass die Messung erfolgreich war und die Messung einen niedrigen, normalen oder hohen Druck ergab.

Ergänzend oder alternativ können somit ferner von einer entfernten Einrichtung, auf welche der Arzt Zugriff hat, z.B. entsprechende, insbesondere auch dem Probanden zugeordnete Parameter, beispielsweise zur Bestimmung von Zeitpunkten zur Durchführung einer jeweiligen Messung, für die Selbsttonometrie-Messung an das Tonometer übertragen werden.

Die im Tonometer angeordneten und durch Aktoren in deren Position und/oder Ausrichtung bewegbaren Teileinheiten, wie also insbesondere die Messeinheit 150 und Düse sowie applikationsspezifisch vorstehende, zur automatischen Blickrichtungserfassung eingesetzte Einheiten und/oder eine Einrichtung, z.B. ein schwenkbar und/oder drehbar angeordneter Düsenkanalabschnitt, an welcher ein angepasster Düsenkopf aufgenommen oder aufnehmbar ist, zur definierten, gezielten Ausrichtung der Impulsbeaufschlagung auf die Hornhaut, sind vorzugsweise an Festkörpergelenkartig ausgebildeten Führungen angehängt. Hierdurch wird ein unerwünschtes Spiel und frühzeitiger Verschleiß der Führungen weitgehend vermieden und folglich eine lange Wartungsfreiheit gewährleistet. Als eine bevorzugte Ausbildung derartiger Festkörpergelenke schlägt die Erfindung Filmscharniere und/oder Federführungen vor.

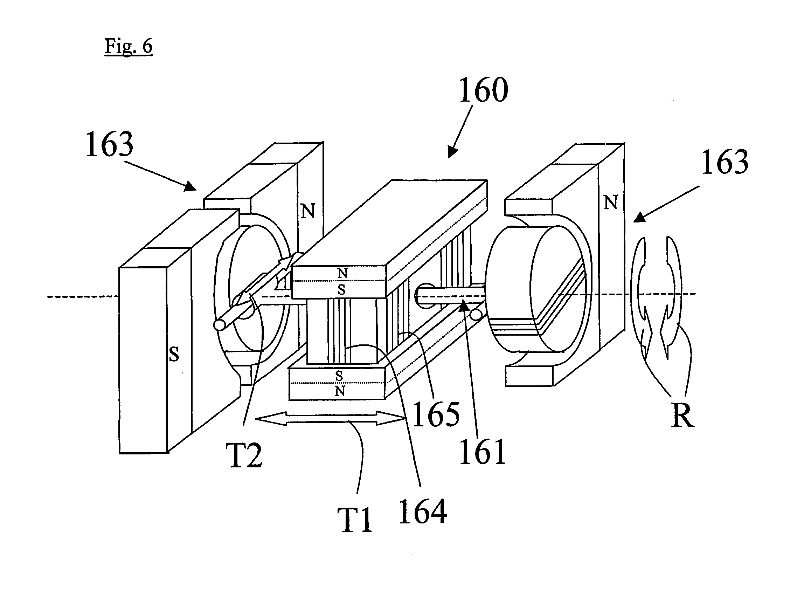

Fig. 6 zeigt stark vereinfacht in teils aufgebrochener Ansicht das Kinematikprinzip eines Positioniersystems zum Bewirken translatorischer und rotatorischer Bewegungen von Sensor- und/oder Mess(teil)einheiten.

Die bei Fig. 6 nicht dargestellte jeweilige Sensor- oder Mess(teil)einheit ist hierbei an einer zentralen Einheit 160 angeordnet, welche einen auf einer Achse 161 in Richtung der Achse beweglich gelagerten Eisenkern 162 mit umgebendem Magnetkreis umfasst, wobei die Achse 161 beidseitig über Drehspulensysteme 163 derart gelagert ist, dass auch eine translatorische Bewegung der Achse 161 senkrecht zu dieser ermöglicht ist.

Über die Drehspulensysteme kann folglich eine Rotation der Sensor- oder Mess(teil)einheit, angezeigt bei Fig. 6 durch die mit "R" gekennzeichneten Richtungspfeilen, um die Achse 161 bewirkt werden, durch welche ein, zweckmäßiger Weise im Wesentlichen vertikaler translatorischer Freiheitsgrad (häufig auch als Y-Richtung bezeichnet) der Sensor- oder Mess(teil)einheit annäherbar ist. Dadurch kann der benötige Bauraum reduziert werden und das Eigengewicht wird in den rotatorischen Lagern getragen. Liegt der Drehpunkt dieser Rotation im Wesentlichen im Schwerpunkt der zu bewegenden der Sensor- oder Mess(teil)einheit, trägt dies wirksam zu einem weiter verbesserten Wirkungsverhältnis und verringerten Energiebedarf bei.

Die verbleibenden zwei im Wesentlichen in horizontaler Richtung verlaufenden Freiheitsgrade der zu bewegenden Sensor- oder Mess(teil)einheit, angezeigt bei Fig. 6 durch die mit "T1" (in einer annähernd parallelen Ebene zur Hornhautoberfläche, häufig auch als X-Richtung bezeichnet) und "T2" (in einer annähernd senkrechten Ebene zur Hornhautoberfläche, häufig auch als Z-Richtung bezeichnet) gekennzeichneten Doppelpfeile, werden basierend auf Fig. 6 durch ein kombiniertes elektrodynamisches Prinzip betrieben, welches statt zwei nur einen Magnetkreis benötigt, wobei zwei Spulen 164 und 165 senkrecht zueinander liegen.

In alternativer Ausführung kann eine Rotation z.B. auch durch einen Schrittmotor bewirkt werden und/oder das translatorische Durchfahren von Freiheitsgraden durch eine entsprechende Anzahl von Linearantrieben.

Insbesondere zur Gewährleistung eines sehr geringen Montageaufwandes können jedoch auch andere Kinematiken verwendet werden, z.B. welche, die im Wesentlichen monolithisch, z.B. durch Spritzguss, Lasertechnik oder Galvanik hergestellt sind.

Eine besondere Ausführungsform des erfindungsgemäßen Tonometers sieht ferner eine Druckbeaufschlagungseinrichtung vor, welche die zur Durchführung der Messung nach entsprechender Ausrichtung notwendige Impuls- bzw. Druckbeaufschlagung mit einem anwendungsspezifisch definierbaren oder vordefinierten linearen und/oder nicht linearen Druckanstieg bereitstellt.

Eine hierfür zweckmäßige Druckbeaufschlagungseinrichtung umfasst z.B. eine, zwischen einer Druckerzeugungseinrichtung und dem Düsenkopf angeordnete Bypass-Einrichtung, welche unter entsprechender zeitbasierter Ansteuerung bestimmte Bypass-Volumen dem in den Düsenkopf einzuleitenden Volumenstrom "integriert".

In weiterer bevorzugter Ausführung ist der Düsenkopf des erfindungsgemäßen mobilen Tonometers in Art einer austauschbaren Einwegeinheit ausgebildet. Selbst bei häufiger Nutzung des Tonometers durch unterschiedliche Probanden ist hierdurch auf einfachste Weise gewährleistbar, dass Tränenflüssigkeit, die durch die NCT-Messung auf das Gerät gespritzt und bei herkömmlichen Tonometern bei der nächsten Messung mit dem Luftstrom mitgerissen werden kann, zumindest vor Weitergabe bzw. Einsatz des Gerätes an einen/einem anderen Probaten ohne zeitintensive, häufig bisher manuell durchzuführende Reinigung entfernbar ist. Hierdurch ist ein wesentlicher Infektionsschutz, insbesondere vor HIV und Hepatitis sichergestellt.

Wie bei Fig. 7 stark vereinfacht dargestellt, umfasst ein solcher, austauschbarer, mit 110 gekennzeichneter Einweg-Düsenkopf 110 bevorzugt einen Zuleitungsabschnitt 111, der zum Zu- bzw. Einleiten "Z" eines Druckbeaufschlagungsmediums, bevorzugt von Druckluft bzw. Luftstößen, mit einem im Tonometer angeordneten Einleitungskanalabschnitt der Düse, z.B. durch einfaches Aufschieben des Zuleitungsabschnittes 111 auf den Einleitungskanalabschnitt, verbindbar ist, eine Düsen-Austrittsöffnung 112, durch welche das Druckbeaufschlagungsmedium in Richtung des Auges austritt "A" und eine die Düsen-Austrittsöffnung 112 und somit den umliegenden Bereich des Düsenkopfes 110 umgebende Schirmung 113 als Spritzschutz.

Insbesondere, wenn die Schirmung 113 auch Bereiche der Messsensorik abdeckt, z.B. wenn der Düsenkopf 110 auf eine Düse 115 der Fig. 1d oder eine in der Einheit 150 der Fig. 4 angeordnete Düse aufsetzbar ist, ist eine Schirmung aus transparentem Material vorgesehen.

Insbesondere im Falle eines Handtonometer ist an diesem darüber hinaus in weiterer zweckmäßiger Ausführung wenigstens ein Haltegriff schwingungsentkoppelt befestigt. Hierdurch wird eine weitere Lagestabilisierung im Vorfeld und während der automatischen Ausrichtung und Feinpositionierung ermöglicht und z.B. Schwankungen/Tremor (z.B. durch Parkinson) des Probanden kompensiert. Eine solche Schwingungsentkopplung ist z.B. mittels der Befestigung über ein passives Feder-Dämpfer-System auf einfache Weise gewährleistbar.

## Patentansprüche

1. Verfahren zur Positionierung der Messachse eines zur Selbsttonometrie ausgebildeten Tonometers, insbesondere eines für eine Non-Contakt-Tonometrie ausgebildeten Tonometers, in Bezug auf ein zu untersuchendes Auge eines Probanden, **dadurch gekennzeichnet, dass** die Blickrichtung des Auges mittels eines integrierten Positionierungssystems automatisch detektiert wird, und zwar durch Detektion der geometrischen Abmessungen der Hornhaut mittels Messung wenigstens eines auf einem optischen Strahlengang basierenden Relativ- und/oder Absolutsignals, und unter Ansprechen auf eine detektierte Blickrichtung des Auges die Messachse des Messsystems vordefiniert im Lot der Hornhaut mittels des Positionierungssystems automatisch justiert und/oder nachgeführt wird.

2. Verfahren nach Anspruch 1, wobei die Detektion kontinuierlich oder periodisch durchgeführt wird und die Messachse nachführend justiert wird.

3. Verfahren nach einem der vorstehenden Ansprüche, bei welchem die Blickrichtungsdetektion mit analoger Signalverarbeitung durchgeführt wird.

4. Verfahren nach einem der vorstehenden Ansprüche, wobei die Detektion mittels Erfassung der Lage, insbesondere Position und/oder Ausrichtung, der Hornhaut durchgeführt wird.

5. Verfahren nach vorstehendem Anspruch, bei welchem die Detektion unter Nutzung von geometrischen Abmessungen der Hornhaut, insbesondere unter Nutzung der Rotationssymmetrie der Hornhaut durchgeführt wird.

6. Verfahren nach vorstehendem Anspruch, bei welchem die geometrischen Abmessungen mittels Messung wenigstens eines auf einem IR-Strahlengang basierenden Relativ- und/oder Absolutsignals detektiert werden.

7. Verfahren nach vorstehendem Anspruch, bei welchem zur Bestimmung von Lagen in Freiheitsgraden des Auges dieses von zwei parallelen Strahlengängen käfigartig umstrahlt wird.

8. Verfahren nach vorstehendem Anspruch, bei welchem zur Bestimmung von Lagen in Freiheitsgraden die Reflexion wenigstens eines Strahlenganges in einem Absolutsignal ausgewertet wird.

9. Verfahren nach einem der vorstehenden Ansprüche, bei welchem zur Bestimmung der Lage der Hornhaut des Auges das relative Verhältnis und die absoluten Pegel wenigstens zweier empfangener Strahlenflüsse ausgewertet werden.

10. Verfahren nach vorstehendem Anspruch, wobei die Messachse im Lot des Hornhautscheitels justiert wird.

11. Verfahren nach einem der vorstehenden Ansprüche, wobei die Messachse in einem definierten Abstand im Lot positioniert wird.

12. Mobiles Tonometer zur Durchführung einer Selbsttonometrie, mit einem integrierten Messsystem (150) zur Durchführung einer berührungsfreien Tonometrie und einem integrierten Positionierungssystem zur Positionierung des Messsystem in Bezug auf ein zu untersuchendes Auge eines Probanden, wobei das Tonometer wenigstens zwei, zur manuellen Grundpositionierung des Tonometers an dem Kopf des Probanden vor dem zu untersuchenden Auge ausgebildeten Anlagebereiche umfasst,
**dadurch gekennzeichnet, dass**
das Positionierungssystem eine Detektionseinrichtung (131, 132, 133) zur automatischen Bestimmung der Lage der Hornhaut des Auges durch Detektion der geometrischen Abmessungen der Hornhaut mittels Messung wenigstens eines auf einem optischen Strahlengang basierenden Relativ- und/oder Absolutsignals umfasst, wobei die Detektionseinrichtung hierzu weinigstens einen optischen Sender zum gerichteten Senden eines Strahls wenigstens teilweise auf die Hornhaut und wenigstens einen optischen Empfänger zum Empfangen wenigstens eines Strahlenanteils des gerichteten Strahls und/oder eines reflektieren Strahls umfasst, und dass das Positionierungssystem eine Ausrichteinrichtung (160) umfasst, die unter Ansprechen auf die Detektion der Lage der Hornhaut automatisch die Messachse des Messsystems vordefinierbar im Lot der Hornhaut justiert und/oder nachführt, insbesondere in einem definierten Abstand im Lot der Hornhaut des Auges positioniert.

13. Tonometer nach vorstehendem Anspruch, wobei die wenigstens zwei Anlagenbereiche in deren Position einstellbar fixierbar sind.

14. Tonometer nach einem der vorstehenden Ansprüche 12 bis 13, ferner **gekennzeichnet durch** eine Peileinrichtung (105, 106) mit einem dem Probanden zugewandten Tubus zur Grundpositionierung der optischen Achse des Auges.

15. Tonometer nach vorstehendem Anspruch, wobei die Peileinrichtung ein mit dem Tubus koaxial angeordnetes optisches Peilmuster (105) derart umfasst, dass das Peilmuster bei axialen Versatz gegenüber der optischen Achse wenigstens teilweise verdeckt oder durch den Tubus nicht sichtbar ist.

16. Tonometer nach einem der Ansprüche 12 bis 15, wobei der wenigstens eine optische Sender ein, IR-Sender (131) und der wenigstens eine optische Empfänger ein, IR-Empfänger (132).

17. Tonometer nach einem der vorstehenden Ansprüche 12 bis 16, ferner **gekennzeichnet durch** wenigstens eine Reflektoreinrichtung, die mit jeweils einem optischen Sender und einem optischen Empfänger zur Bildung eines geometrische Abmessungen der Hornhaut markierenden Strahlengangs zusammenwirken.

18. Tonometer nach einem der vorstehenden Ansprüche 12 bis 17, wobei das Positionierungssystem eine Steuer-/Regeleinheit und Führungen zum Justieren wenigstens eines optischen Senders, wenigstens eines optischen Empfängers und/oder wenigstens einer Reflektoreinrichtung und/oder die Ausrichteinrichtung eine Steuer-/Regeleinheit und Führungen zum Justieren des Messsystems umfasst oder mit dieser zusammenwirkt.

19. Tonometer nach einem der vorstehenden Ansprüche 12 bis 18, **gekennzeichnet durch** zwei optische Sender und zwei optische Empfänger mit jeweils zugeordneter Reflektoreinrichtung zur Bildung eines, die geometrische Hornhautabmessung markierenden Strahlengangkäfigs.

20. Tonometer nach einem der vorstehenden Ansprüche 12 bis 19, **gekennzeichnet durch** einen optischen Sender und einen optischen Empfänger mit zugeordneter Reflektoreinrichtung zur Bewirkung einer, die geometrische Hornhautabmessung markierenden Strahlengangabtastung.

21. Tonometer nach einem der vorstehenden Ansprüche 12 bis 20, **gekennzeichnet durch** wenigsten einen optischen Sender und einen optischen Empfänger, die für eine auf der Reflexion eines Strahlengangs basierende Auswertung von Lagen in Freiheitsgraden zur Bestimmung der Position und/oder Ausrichtung der Hornhaut positionierbar sind.

22. Tonometer nach vorstehendem Anspruch,
**gekennzeichnet durch** wenigsten einen optischen Sender und einer Mehrzahl von um den Sender angeordneten, vorzugsweise vier rotationssymmetrisch um den Sender angeordneten, optischen Empfängern, insbesondere Photodioden.

23. Tonometer nach einem der vorstehenden Ansprüche 12 bis 22, ferner **gekennzeichnet durch** Festkörpergelenkführungen, zum Bewegen von justierbar angeordneten (Teil-)Einheiten des Positionierungssystems und des Messsystems.

24. Tonometer nach vorstehendem Anspruch, ferner **dadurch gekennzeichnet, dass** die Festkörpergelenkführungen Filmscharniere und/oder Federführungen umfassen.

25. Tonometer nach vorstehendem Anspruch, ferner **dadurch gekennzeichnet, dass** Festkörpergelenkführungen zum Bewegen einer justierbar angeordneten Einrichtung Teil einer monolithisch, insbesondere durch Spritzguss-, Galvanik- oder Lasertechnik, hergestellten Kinematik (160) sind.

26. Tonometer nach einem der vorstehenden Ansprüche 12 bis 25, ferner **gekennzeichnet durch** eine Translations- und/oder Rotationskinematik, insbesondere Festkörpergelenkführungen umfassend, zum Bewegen einer justierbar angeordneten Einrichtung.

27. Tonometer nach einem der beiden vorstehenden Ansprüche, ferner **dadurch gekennzeichnet, dass** Festkörpergelenkführungen und/oder wenigstens eine Kinematik Aktor-betrieben sind.

28. Tonometer nach einem der vorstehendem Ansprüche 12 bis 27, ferner **dadurch gekennzeichnet, dass** Führungen, insbesondere Festkörpergelenkführungen zur Positionierung von Sensor- und/oder Mess(teil)einheiten in im Wesentlichen drei senkrecht zueinander verlaufenden Freiheitsgraden ausgebildet sind.

29. Tonometer nach vorstehendem Anspruch, ferner
**dadurch gekennzeichnet, dass** zur Positionierung in wenigstens einem Freiheitsgrad eine rotatorische Bewegung, insbesondere mit einer durch den Schwerpunkt der zu bewegenden Masse verlaufenden Drehachse, vorgesehen ist.

30. Tonometer nach einem der vorstehenden Ansprüche 12 bis 29, ferner **gekennzeichnet durch** eine Druckbeaufschlagungseinrichtung zum definierten Zuführen eines Druckbeaufschlagungsmediums, insbesondere von Luft, auf das Auge.

31. Tonometer nach einem der vorstehenden Ansprüche 12 bis 30, ferner **gekennzeichnet durch** eine Druckbeaufschlagungseinrichtung zum definierten Zuführen von Luft auf das Auge welche einen austauschbaren Düsenkopf (110) aufnimmt.

32. Tonometer nach vorstehendem Anspruch, ferner **dadurch gekennzeichnet, dass** der Düsenkopf (110) eine die Düsenöffnung (112) in radialer Richtung zur Zuführrichtung umgebende Schirmung (113) umfasst.

33. Tonometer nach einem der beiden vorstehenden Ansprüche, ferner **dadurch gekennzeichnet, dass** der Düsenkopf auf einer beweglich angeordneten Einrichtung zum Einleiten des Druckbeaufschlagungsmediums in den Düsenkopf angeordnet ist.

34. Tonometer nach vorstehendem Anspruch, ferner **dadurch gekennzeichnet, dass** die bewegliche Einrichtung mit der Ausrichteinrichtung des Positionierungssystems gekoppelt ist.

35. Tonometer nach einem der vorstehenden Ansprüche 12 bis 34, welches als Anlagebereiche zum Ansetzen ein Nasenbein, eine Schläfe und/oder einen Wangenknochen entsprechend geformte und/oder formbare Stützbereiche umfasst.

36. Tonometer nach einem der vorstehenden Ansprüche 12 bis 35, welches Brillen-artig oder Helm-artig ausgebildet ist.

37. Tonometer nach einem der vorstehenden Ansprüche 12 bis 35, welches als Handtonometer ausgebildet ist.

38. Tonometer nach vorstehendem Anspruch, bei welchem das Positionierungssystem und Messsystem in einem Gehäuse angeordnet sind, mit welchem schwingungsentkoppelt wenigstens ein Haltegriff verbunden ist.

39. Tonometer nach vorstehendem Anspruch, bei welchem das Gehäuse und der wenigstens eine Haltegriff über ein passives Feder-Dämpfer-System verbunden sind.

40. Tonometer nach einem der vorstehenden Ansprüche 12 bis 39, ferner umfassend eine Datenverarbeitungseinrichtung oder eine mit einer Datenverarbeitungseinrichtung korrespondierende Sende- und/oder Empfangseinrichtung.

41. Verfahren zur Positionierung der Messachse eines zur Selbsttonometrie ausgebildeten Tonometers, insbesondere eines für eine Non-Contakt-Tonometrie ausgebildeten Tonometers, in Bezug auf ein zu untersuchendes Auge eines Probanden, **dadurch gekennzeichnet, dass** die Lage der Hornhaut des Auges mittels eines integrierten Positionierungssystems automatisch detektiert wird, und zwar durch Detektion der geometrischen Abmessungen der Hornhaut mittels Messung wenigstens eines auf einem optischen Strahlengang basierenden Relativ- und/oder Absolutsignals, und unter Ansprechen auf eine detektierte Lage der Hornhaut des Auges die Messachse des Messsystems vordefiniert im Lot der Hornhaut mittels des Positionierungssystems automatisch justiert und/oder nachgeführt wird.

## Claims

1. Method for positioning the measuring axis of a tonometer formed for self tonometry, in particular of a tonometer formed for contactless tonometry, in relation to a subject's eye which is to be examined, **characterised in that** the line of vision of the eye is automatically detected by means of an integrated positioning system, and in particular by detecting the geometric dimensions of the cornea by measuring at least one relative and/or absolute signal based on an optical beam path, and in response to a detected line of vision of the eye, the measuring axis of the measuring system is automatically adjusted and/or followed in a predefined manner perpendicular to the cornea by means of the positioning system.

2. Method as claimed in claim 1, wherein the detection is carried out continuously or periodically and the measuring axis is adjusted by being followed.

3. Method as claimed in any one of the preceding claims, wherein the line of vision is detected by analogue signal processing.

4. Method as claimed in any one of the preceding claims, wherein the detection is effected by detecting the location, in particular the position and/or orientation of the cornea.

5. Method as claimed in the preceding claim, wherein the detection is carried out using geometric dimensions of the cornea, in particular using the rotational symmetry of the cornea.

6. Method as claimed in the preceding claim, wherein the geometric dimensions are detected by measuring at least one relative and/or absolute signal based on an IR beam path.

7. Method as claimed in the preceding claim, wherein in order to determine locations in degrees of freedom of the eye, this eye has two parallel beam paths radiated around it in a cage-like manner.

8. Method as claimed In the preceding claim, wherein in order to determine locations in degrees of freedom the reflection of at least one beam path in an absolute signal is evaluated.

9. Method as claimed in any one of the preceding claims, wherein in order to determine the location of the cornea of the eye the relative ratio and the absolute level of at least two received beam flows are evaluated.

10. Method as claimed in the preceding claim, wherein the measuring axis is adjusted perpendicular to the corneal vertex.

11. I Method as claimed in any one of the preceding claims, wherein the measuring axis is positioned perpendicularly at a defined distance.

12. Mobile tonometer for carrying out self tonometry, having an integrated measuring system (150) for carrying out contactless tonometry and an integrated positioning system for positioning the measuring system with respect to a subject's eye which is to be examined, wherein the tonometer has at least two contact regions formed for manual basic positioning of the tonometer on the head of the subject in front of the eye to be examined **characterised in that** the positioning system has a detection device (131,132,133) for automatic determination of the location of the cornea of the eye by detecting the geometric dimensions of the cornea by measuring at least one relative and/or absolute signal based on an optical beam path, wherein, for this purpose, the detection device has at least one optical emitter for directed emission of a beam at least partially onto the cornea and at least one optical receiver for receiving at least a portion of the directed beam and/or a reflected beam, and that the positioning system has an orientation device (160) which, in response to the detection of the location of the cornea, automatically adjusts and/or follows the measuring axis of the measuring system in a predefinable manner perpendicular to the cornea, in particular positioned at a defined distance perpendicular to the cornea of the eye.

13. Tonometer as claimed in the preceding claim, wherein the position of the at least two contact regions can be adjustably fixed.

14. Tonometer as claimed in any one of the preceding claims 12 to 13, further **characterised by** a bearing device (105, 106) with a lens tube facing the subject for basic positioning of the optical axis of the eye.

15. Tonometer as claimed in the preceding claim, wherein the bearing device has an optical bearing pattern (105) - disposed coaxial to the lens tube - such that, in the event of axial displacement with respect to the optical axis, the bearing pattern is at least partially concealed or cannot be soon through the lens tube.

16. Tonometer as claimed in any one of claims 12 to 15, wherein the at least one optical emitter is a IR emitter (131) and the at least one optical receiver is an IR receiver (132).

17. Tonometer as claimed in any one of the preceding claims 12 to 16, further **characterised by** at least one reflector device which cooperates with an optical emitter and an optical receiver respectively to form a beam path marking geometric dimensions of the cornea.

18. Tonometer as claimed in any one of the preceding claims 12 to 17, wherein the positioning system has a control/regulating unit and guides for adjustment of at least one optical emitter, of at least one optical receiver and/or of at least one reflector device, and/or the orientation device has a control/regulating unit and guides for adjustment of the measuring system, or cooperates therewith.

19. Tonometer as claimed in any one of the preceding claims 12 to 18, **characterised by** two optical emitters and two optical receivers each with an associated reflector device for formation of a beam path cage marking the geometric corneal dimension,

20. Tonometer as claimed in any one of the preceding claims 12 to 19, **characterised by** an optical emitter and an optical receiver with an associated reflector device for effecting beam path sampling, marking the geometric corneal dimension.

21. Tonometer as claimed in any one of the preceding claims 12 to 20, **characterised by** at least one optical emitter and an optical receiver, which can be positioned for an evaluation - based on the reflection of a beam path - of locations in degrees of freedom in order to determine the position and/or orientation of the cornea.

22. Tonometer as claimed in the preceding claim, **characterised by** at least one optical emitter and a plurality of optical receivers, in particular photodiodes, disposed around the emitter, preferably being four in number, disposed with rotational symmetry around the emitter.

23. Tonometer as claimed in any one of the preceding claims 12 to 22, further **characterised by** solid body joint guides for movement of adjustably disposed (sub-)units of the positioning system and of the measuring system.

24. Tonometer as claimed in the preceding claim, further **characterised in that** the solid body joint guides include film hinges and/or spring guides.

25. Tonometer as claimed in the preceding claim, further **characterised in that** solid body joint guides for movement of an adjustably disposed device are part of a kinematic arrangement (160) produced monolithically, in particular by injection moulding, electroplating or laser technology.

26. Tonometer as claimed in any one of the preceding claims 12 to 25, further **characterised by** a translational, and/or rotational kinematic arrangement, in particular comprising solid body joint guides for movement of an adjustably disposed device.

27. Tonometer as claimed in any one of the two preceding claims, further **characterised in that** solid body joint guides and/or at least one kinematic arrangement are driven by an actuator.

28. Tonometer as claimed in any one of the preceding claims 12 to 27, further **characterised in that** guides, in particular solid body joint guides, are formed for positioning of sensor and/or measuring (sub-)units in substantially three degrees of freedom extending perpendicular to each other.

29. Tonometer as claimed in the preceding claim, further **characterised in that** a rotational movement, in particular with an axis of rotation extending through the centre of gravity of the mass to be moved, is provided for positioning in at least one degree of freedom.

30. Tonometer as claimed in any one of the preceding claims 12 to 29, further **characterised by** a pressure application device for defined supply of a pressure application medium, in particular air, to the eye.

31. Tonometer as claimed in any one of the preceding claims 12 to 30, further **characterised by** a pressure application device for defined supply of air to the eye, which receives an exchangeable nozzle head (110).

32. Tonometer as claimed in the preceding claim, further **characterised in that** the nozzle head (110) has a screen (113) surrounding the nozzle opening (112) in the radial direction with respect to the supply device.

33. Tonometer as claimed in any one of the two preceding claims, further **characterised in that** the nozzle head is disposed on a moveably disposed device for introducing the pressure application medium into the nozzle head.

34. Tonometer as claimed in the preceding claim, further **characterised in that** the moveable device is coupled to the orientation device of the positioning system.

35. Tonometer as claimed in any one of the preceding claims 12 to 34, which, as contact regions for placement purposes, has support regions which are and/or can be formed corresponding to a nasal bone, a temple and/or a cheekbone.

36. Tonometer as claimed in any one of the preceding claims 12 to 35, which is formed in a spectacle-like or helmet-like manner.

37. Tonometer as claimed in any one of the preceding claims 12 to 35, which is formed as a hand-held tonometer.

38. Tonometer as claimed in the preceding claim, wherein the positioning system and measuring system are disposed in a housing, to which at least one handle is connected in a vibration-decoupled manner.

39. Tonometer as claimed in the preceding claim, wherein the housing and the at least one handle are connected via a passive spring-damper system.

40. Tonometer as claimed in any one of the preceding claims 12 to 39, further comprising a data processing device or an emitting and/or receiving device communicating with a data processing device.

41. Method for positioning the measuring axis of a tonometer formed for selftonometry, in particular of a tonometer formed for contactless tonometry, in relation to a subject's eye which is to be examined, **characterised in that** the location of the cornea of the eye is automatically detected by means of an integrated positioning system, in particular by detecting the geometric dimensions of the cornea by measuring at least one relative and/or absolute signal based on an optical beam path, and, in response to a detected location of the cornea of the eye, the measuring axis of the measuring system is automatically adjusted and/or followed in a predefined manner perpendicular to the cornea by means of the positioning system.

## Revendications

1. Procédé pour le positionnement de l'axe de mesure d'un tonomètre conçu pour l'autotonométrie, en particulier d'un tonomètre conçu pour une tonométrie sans contact, par rapport à un oeil à examiner d'un sujet, **caractérisé en ce que** la direction du regard de l'oeil est détectée automatiquement au moyen d'un système de positionnement intégré, et ce par détection des dimensions géométriques de la cornée par mesure d'au moins un signal relatif et/ou d'un signal absolu basé sur la trajectoire de faisceau optique, et est ajustée et/ou asservie automatiquement à la verticale de la cornée au moyen du système de positionnement, en prédéfinissant l'axe de mesure du système de mesure en réaction à une direction du regard détectée de l'oeil.

2. Procédé selon la revendication 1, la détection étant effectuée de façon continue ou périodique et l'axe de mesure étant ajusté de façon asservie.

3. Procédé selon l'une quelconque des revendications précédentes, dans lequel la détection de la direction du regard est effectuée avec un traitement de signal analogique.

4. Procédé selon l'une quelconque des revendications précédentes, la détection étant effectuée par détection de l'emplacement, en particulier de la position et/ou de l'orientation, de la cornée.

5. Procédé selon la revendication précédente, dans lequel la détection est effectuée en utilisant des dimensions géométriques de la cornée, en particulier en utilisant la symétrie de rotation de la cornée.

6. Procédé selon la revendication précédente, dans lequel les dimensions géométriques sont détectées par mesure d'au moins un signal relatif et/ou d'un signal absolu basé sur une trajectoire de faisceau infrarouge.

7. Procédé selon la revendication précédente, dans lequel, pour déterminer des emplacements dans des degrés de liberté de l'oeil, celui-ci est entouré à la façon d'une cage par deux trajectoires de faisceau parallèles.

8. Procédé selon la revendication précédente, dans lequel, pour déterminer des emplacements dans des degrés de liberté, la réflexion d'au moins une trajectoire de faisceau est analysée dans un signal absolu.

9. Procédé selon l'une quelconque des revendications précédentes, dans lequel, pour déterminer l'emplacement de la cornée de l'oeil, le rapport relatif et les niveaux absolus d'au moins deux flux de faisceau reçus sont analysés.

10. Procédé selon la revendication précédente, l'axe de mesure étant ajusté à la verticale du sommet de la cornée.

11. Procédé selon l'une quelconque des revendications précédentes, l'axe de mesure étant positionné à une distance définie à la verticale.

12. Tonomètre mobile pour la réalisation d'une autotonométrie, comprenant un système de mesure (150) intégré pour la réalisation d'une tonométrie sans contact et un système de positionnement intégré pour le positionnement du système de mesure par rapport à un oeil à examiner d'un sujet, le tonomètre comprenant au moins deux zones d'appui conçues pour le positionnement de base manuel du tonomètre sur la tête du sujet devant l'oeil à examiner,
**caractérisé en ce que** :
le système de positionnement comprend un système de détection (131, 132, 133) pour la détermination automatique de l'emplacement de la cornée de l'oeil par détection des dimensions géométriques de la cornée au moyen de la mesure d'au moins un signal relatif et/ou d'un signal absolu basé sur une trajectoire de faisceau optique, le dispositif de détection comprenant à cet effet au moins un émetteur optique pour l'envoi dirigé d'un faisceau au moins partiellement sur la cornée et au moins un récepteur optique pour la réception d'au moins une fraction de faisceau du faisceau dirigé et/ou d'un faisceau réfléchi, et **en ce que** le système de positionnement comprend un dispositif d'orientation (160), qui ajuste et/ou asservit automatiquement l'axe de mesure du système de mesure de façon prédéfinissable à la verticale de la cornée, en réaction à la détection de l'emplacement de la cornée, en particulier le positionne à une distance définie à la verticale de la cornée de l'oeil.

13. Tonomètre selon la revendication précédente, les au moins deux zones d'appui pouvant être fixées de façon réglable dans leur position.

14. Tonomètre selon l'une quelconque des revendications 12 et 13 précédentes, caractérisé également par un dispositif de relèvement (105, 106) comprenant un tube, tourné vers le sujet, pour le positionnement de base de l'axe optique de l'oeil.

15. Tonomètre selon la revendication précédente, le dispositif de relèvement comprenant un modèle de relèvement (105) optique, disposé sur le même axe que le tube, de telle sorte que le modèle de relèvement est au moins en partie masqué dans le cas d'un déport axial par rapport à l'axe optique ou n'est pas visible par le tube.

16. Tonomètre selon l'une quelconque des revendications 12 à 15, l'au moins un émetteur optique étant un émetteur infrarouge (131), et l'au moins un récepteur optique étant un récepteur infrarouge (132).

17. Tonomètre selon l'une quelconque des revendications 12 à 16 précédentes, caractérisé également par au moins un dispositif réflecteur, qui coopère avec respectivement un émetteur optique et un récepteur optique pour la formation d'une trajectoire de faisceau marquant des dimensions géométriques de la cornée.

18. Tonomètre selon l'une quelconque des revendications 12 à 17 précédentes, le système de positionnement comprenant une unité de commande et de réglage et des guides pour l'ajustage d'au moins un émetteur optique, d'au moins un récepteur optique et/ou d'au moins un dispositif réflecteur et/ou le dispositif d'orientation comprenant une unité de commande/réglage et des guides pour l'ajustage du système de mesure ou coopérant avec celui-ci.

19. Tonomètre selon l'une quelconque des revendications 12 à 18 précédentes, **caractérisé par** deux émetteurs optiques et deux récepteurs optiques comprenant à chaque fois le dispositif réflecteur associé pour la formation d'une cage de trajectoire de faisceau marquant la dimension géométrique de la cornée.

20. Tonomètre selon l'une quelconque des revendications 12 à 19 précédentes, **caractérisé par** un émetteur optique et un récepteur optique avec dispositif réflecteur associé pour entraîner un balayage de trajectoire de faisceau marquant la dimension géométrique de la cornée.

21. Tonomètre selon l'une quelconque des revendications 12 à 20 précédentes, **caractérisé par** au moins un émetteur optique et un récepteur optique, qui peuvent être positionnés pour une analyse d'emplacements, basée sur la réflexion d'une trajectoire de faisceau, dans des degrés de liberté pour déterminer la position et/ou l'orientation de la cornée.

22. Tonomètre selon la revendication précédente, **caractérisé par** au moins un émetteur optique et une pluralité de récepteurs optiques, disposés autour de l'émetteur, de préférence quatre disposés avec une symétrie de rotation autour de l'émetteur, en particulier des photodiodes.

23. Tonomètre selon l'une quelconque des revendications 12 à 22 précédentes, caractérisé également par des guides articulés à corps solide, pour le déplacement d'unités (partielles) disposées de façon ajustable du système de positionnement et du système de mesure.

24. Tonomètre selon la revendication précédente, caractérisé également en ce que les guides articulés à corps solide comprennent des charnières à film et/ou des guides à ressort.

25. Tonomètre selon la revendication précédente, caractérisé également en ce que des guides articulés à corps solide pour le déplacement d'un dispositif disposé de façon ajustable font partie d'une cinématique (160) établie de façon monolithe, en particulier par technique de moulage par injection, technique de galvanisation ou technique de laser.

26. Tonomètre selon l'une quelconque des revendications 12 à 25 précédentes, caractérisé également par une cinématique de translation et/ou une cinématique de rotation, comprenant en particulier des guides articulés à corps solide, pour le déplacement d'un dispositif disposé de façon ajustable.

27. Tonomètre selon l'une quelconque des deux revendications précédentes, caractérisé également en ce que des guides articulés à corps solide et/ou au moins une cinématique sont exploités par actionneur.

28. Tonomètre selon l'une quelconque des revendications 12 à 27 précédentes, caractérisé également en ce que des guides, en particulier des guides articulés à corps solide, sont conçus pour le positionnement d'unités (partielles) de détection et/ou de mesure dans principalement trois degrés de liberté agencés perpendiculairement entre eux.

29. Tonomètre selon la revendication précédente, caractérisé également en ce que, pour le positionnement dans au moins un degré de liberté, il est prévu un mouvement de rotation, en particulier avec un axe de rotation passant par le centre de gravité de la masse à déplacer.

30. Tonomètre selon l'une quelconque des revendications 12 à 29 précédentes, caractérisé également par un dispositif de sollicitation en pression pour l'arrivée définie d'un fluide de sollicitation en pression, en particulier d'air, sur l'oeil.

31. Tonomètre selon l'une quelconque des revendications 12 à 30 précédentes, caractérisé également par un dispositif de sollicitation en pression pour l'arrivée définie d'air sur l'oeil qui reçoit une tête de buse (110) interchangeable.

32. Tonomètre selon la revendication précédente, caractérisé également en ce que la tête de buse (110) comprend un blindage (113) entourant l'ouverture de buse (112) dans la direction radiale par rapport au sens d'arrivée.

33. Tonomètre selon l'une quelconque des deux revendications précédentes, caractérisé également en ce que la tête de buse est disposée sur un système, disposé de façon mobile, pour l'introduction du fluide de sollicitation en pression dans la tête de buse.

34. Tonomètre selon la revendication précédente, caractérisé également en ce que le dispositif mobile est couplé avec le dispositif d'orientation du système de positionnement.

35. Tonomètre selon l'une quelconque des revendications 12 à 34 précédentes, qui comprend comme zones d'appui des zones de soutien formées de façon appropriée et/ou formables pour mettre en place un os de nez, une tempe et/ou un os de joue.

36. Tonomètre selon l'une quelconque des revendications 12 à 35 précédentes, qui est conçu à la façon d'une lunette ou d'un casque.

37. Tonomètre selon l'une quelconque des revendications 12 à 35 précédentes, qui est conçu comme tonomètre manuel.

38. Tonomètre selon la revendication précédente, dans lequel le système de positionnement et le système de mesure sont disposés dans un boîtier, avec lequel au moins une poignée de maintien est reliée de façon dissociée des vibrations.

39. Tonomètre selon la revendication précédente, dans lequel le boîtier et l'au moins une poignée de retenue sont reliés par un système d'amortisseur à ressort passif.

40. Tonomètre selon l'une quelconque des revendications 12 à 39 précédentes, comprenant également un dispositif de traitement de données ou un dispositif d'émission et/ou de réception correspondant à un dispositif de traitement de données.

41. Procédé pour le positionnement de l'axe de mesure d'un tonomètre conçu pour l'autotonométrie, en particulier d'un tonomètre conçu pour une tonométrie sans contact, par rapport à un oeil à examiner d'un sujet, **caractérisé en ce que** l'emplacement de la cornée de l'oeil est détecté automatiquement au moyen d'un système de positionnement intégré, et ce par détection des dimensions géométriques de la cornée par la mesure d'au moins un signal relatif et/ou d'un signal absolu basé sur une trajectoire de faisceau optique, et l'axe de mesure du système de mesure est ajusté et/ou asservi automatiquement à la verticale de la cornée au moyen du système de positionnement de façon prédéfinie en réaction à un emplacement détecté de la cornée de l'oeil.
